# EUROPEAN PATENT APPLICATION

(11) **EP 4 585 165 A1**
(43) Date of publication of application: **16.07.2025**
(21) Application number: 22957643.4
(22) Date of filing: 05.09.2022
(51) Int. Cl.: A61B 17/04

(54) **CAPSULE NEEDLE AND THREAD MODULE**

(71) Applicant: MATRIXLabs Medical Inc., New Taipei City, 244019 (TW)
(72) Inventor: TU, Fung-Chao, NewTaipei City, Taiwan 22060 (TW); WU, Wen-Yih, Taipei City, Taiwan 10550 (TW); SUNG, Ching Chun, NewTaipei City, Taiwan 220 (TW); WANG, Chung Chih, NewTaipei City, Taiwan 220 (TW); SU, Jimmy Chunmin, Kaohsiung City, Taiwan 81356 (TW); KUO, Chia-Sheng, NewTaipei City, Taiwan 244 (TW); POLAK, Pim David, 3743 Baarn (NL)
(74) Representative: Cabinet Chaillot
(86) International application number: PCT/CN2022/117089
(87) International publication number: WO 2024/050668

(57) **Abstract**

A capsule knotting module has an operating member (10) and a knotting member (20). With a suture (A), a pre-knot (A1) and a sliding loop (A2) are formed at a front end of the knotting member (20). When in use, the pre-knot (A1) is tied to form a secure knot at a front end of the knotting member (20) to achieve the purpose of suturing and fixing a tissue wound.

## Description

### FIELD OF INVENTION

The present invention relates to a knotting assembly, and more particularly to a capsule knotting module.

### BACKGROUND OF THE INVENTION

With the advancement of modern medical technology, surgical techniques have also evolved toward minimally invasive approaches, such as small incisions or minimally invasive procedures. These not only facilitate rapid and effective wound healing but also help reduce and improve post-operative scarring. In a conventional knot-tying process, when a surgeon aims to close a wound, they typically use a handheld surgical needle with an attached suture. The tissue on both sides of the wound is sutured and tightened, forming a secure knot to anchor the end of the suture.

However, it is complex and difficult to perform the conventional knot-tying process manually by to wrap the surgical suture and to form the secure knot during a minimally invasive surgery, which usually results in a poor quality of the secure knot. In addition, it is inefficient to tie multiple secure knots by the conventional method and it increases the risk of wound dehiscence while the secure knots are under poor quality.

### SUMMARY OF THE INVENTION

To overcome the issues associated with the conventional knotting techniques used in surgical wound closures, such as the inefficiency of repeated operations to form multiple secure knots and the risk of wound dehiscence affecting the patient's recovery efficiency.

The present invention provides a capsule knotting module comprising an operating member and a knotting member. The operating member is arranged at a rear end of the capsule knotting module. The knotting member is arranged at a front end of the capsule knotting module and has a through hole formed through the knotting member. A shaft extends through the through hole and protrudes from the front end of the capsule knotting module. A suture has two ends. One of the two ends of the suture is connected to a part of the operating member, and the other end of the suture extends through the through hole and the shaft and extends toward and is exposed from the front end of the capsule knotting module.

Wherein, at least a part of a knotting end of the suture is wrapped around an outer circumference of the shaft and forms a pre-knot, and a sliding loop is formed between the shaft and the pre-knot. A size of the sliding loop is varied according to a distance between the operating member and the knotting member.

Wherein, the knotting member comprises a connecting portion detachably connected with the operating member.

Wherein, the connecting portion comprises a cavity defined in a rear end of the knotting member, wherein a groove is defined annually in an inner wall of the cavity, and the operating member comprises two connecting ribs extending toward the cavity and spaced from each other, each connecting rib having a raised part diametrically protruding from a front end of the connecting rib at a position corresponding to the groove and engaged with the groove in a concave-convex manner.

Wherein, a limiting hole is defined in a front end of the knotting member. A limiting suture extends toward a front end of the knotting member and out from the limiting hole. A limiting loop is formed on at least a part of the limiting suture protruding out of the limiting hole. At least a part of the limiting loop is hooked with the pre-knot or the sliding loop.

Wherein, the through hole and the limiting hole are parallel to each other, formed through the knotting member, and communicate with the cavity. The limiting suture is folded and held in the limiting hole. Two ends of the limiting suture extend toward the rear end of the limiting hole and into the cavity. The limiting loop formed by the folded limiting suture extends toward a front end of the limiting hole and is hooked with the pre-knot. When the operating member is assembled with the connecting portion of the knotting member, at least a part of the operating member presses the two ends of the limiting suture at the connecting portion.

Wherein, the knotting member comprises a stop portion formed on the knotting member, and the stop portion comprises an extension section formed on a surface of the knotting member and extending toward the front end of the capsule knotting module and a stopping section protruding in a diametrical direction of the through hole from a front end of the extension section, wherein a rear end of the shaft protruding out from the through hole abuts with an end surface of the stopping section.

Wherein, the capsule knotting module further comprises a housing covering at least a part of a front end of the operating member and at least a part of a rear end of the knotting member. A pull rod is formed on the front end of the operating member, one of the ends of the suture is bound on the pull rod. An operating portion is formed on a rear end of the operating member and protrudes out of the housing. The operating portion is connected with the pull rod by an extending arm. The connecting portion is formed on the rear end of the knotting member at a position adjacent to the operating portion. At least a part of the connecting portion is overlapped with at least a part of the front end of the operating member in the housing. A front end of the operating portion is slidable relative to the connecting portion in the housing. A notch is defined in a surface of the connecting portion corresponding to the operating member.

Wherein, the operating member comprises a connecting rib protruding from the operating portion. The extending arm and the connecting rib are spaced from each other. A raised part protrudes from the connecting rib on a front end extending into the housing and facing the groove.

Wherein, the suture exposed at the front end of the capsule knotting module is connected with a surgical needle.

Wherein, the operating member comprises the pull rod formed diametrically on the operating member, one of the ends of the suture is bound on the pull rod.

Wherein, the two connecting ribs protrude respectively from two ends of the pull rod and extend toward the front end of the capsule knotting module, the suture extends through the through hole, the cavity, and a space between the two connecting ribs and is bound on the pull rod.

Wherein, a tying portion is defined on at least a part of the shaft protruding from the through hole. A length of the tying portion is varied according to a relative position of the shaft and the through hole.

Wherein, an outer diameter of the knotting member is tapered from the rear end to the front end to form a conical shape.

When the capsule knotting module is in use, a user aligns the front end of the knotting member with an intended suture site and the surgical needle is passed through the intended suture site. The surgical needle with the suture is passed through the sliding loop. Once suturing is complete, the operating member is moved away from the knotting member to allow the suture to be pulled by the operating member and to make the sliding loop be shrunk until it adheres to the outer circumference of the shaft.

Continuously pulling the operating member and/or simultaneously pulling the surgical needle, a tension is generated at two ends of the suture to make the pre-knot be escaped from the shaft. Thus, the sliding loop is tied with the suture extending through the sliding loop to form a secure knot.

Compared with the current technology, the advantage of the present invention comprises:
The capsule knotting module of the present invention has a simple structure, and can be designed in various suture size as needed. It can be miniaturized for fit a minimally invasive surgery, and assisting the operators in completing suturing and knotting procedures seamlessly in narrow spaces, optimizing the surgical time and the quality of knotting. Furthermore, by extending its length while minimizing suture usage, the capsule knotting module reduces costs effectively.

The stop portion assists operators in aligning the capsule knotting module with the intended suture site while preventing the shaft from directly contacting the suture and avoiding contamination by blood or tissue fluid. Additionally, the stop portion effectively prevent the pre-knot from slipping from the shaft unexpectedly.

Particularly when the shaft is disposed in the through hole, during the formation of the secure knot, the operator must apply sufficient tension by pulling the operating member and /or the surgical needle. This tension causes the pre-knot to push against and deform the stop portion, allowing it to slip from the shaft. During this process, the stop portion can prevent the pre-knot from slipping off from the sleeve shaft in advance, thereby preventing an undesirable knot location or poor knot quality that could result in looseness or failure.

### IN THE DRAWINGS

Fig. 1 is a perspective view of a first preferred embodiment of a capsule knotting module in accordance with the present invention;
Fig. 2 is an exploded perspective view of the capsule knotting module in Fig. 1;
Fig. 3a is a first operating side view in partial section of the capsule knotting module in Fig. 1;
Fig. 3b is a second operating side view in partial section of the capsule knotting module in Fig. 1;
Fig. 4 is a perspective view in partial section of a second preferred embodiment of a capsule knotting module in accordance with the present invention;
Fig. 5 is a perspective view of a third preferred embodiment of a capsule knotting module in accordance with the present invention;
Fig. 6 is an exploded perspective view of the capsule knotting module in Fig. 5;
Fig. 7a is a first operating side view in partial section of the capsule knotting module in Fig. 5; and
Fig. 7b is a second operating side view in partial section of the capsule knotting module in Fig. 5.

### Symbol description:

10 operating member
11 connecting rib
111 raised part
12 pull rod
13 operating portion
131 extending arm
20 knotting member
21 through hole
22 shaft
221 tying portion
23 connecting portion
231 cavity
232 groove
235 notch
24 limiting hole
25 stop portion
251 extension section
252 stopping section
26 holding space
30 housing
A suture
A1 pre-knot
A2 sliding loop
B surgical needle
C limiting suture
C1 limiting loop

### DETAILED DESCRIPTION OF THE PREFERRED EMBODIMENTS

In order to make purposes, technical solutions, and advantages of the present invention to be clearer, the following content provides some preferred embodiments in accordance with the present invention in cooperation with the drawings to clearly and completely describe the techniques of the present invention. The following embodiments are only a part of embodiments of the present invention, but not all the embodiments of the present invention. Based on the following embodiments of the present invention, the person having an ordinary skill in the art can get embodiments without any inventive work that are all the embodiments of the present invention.

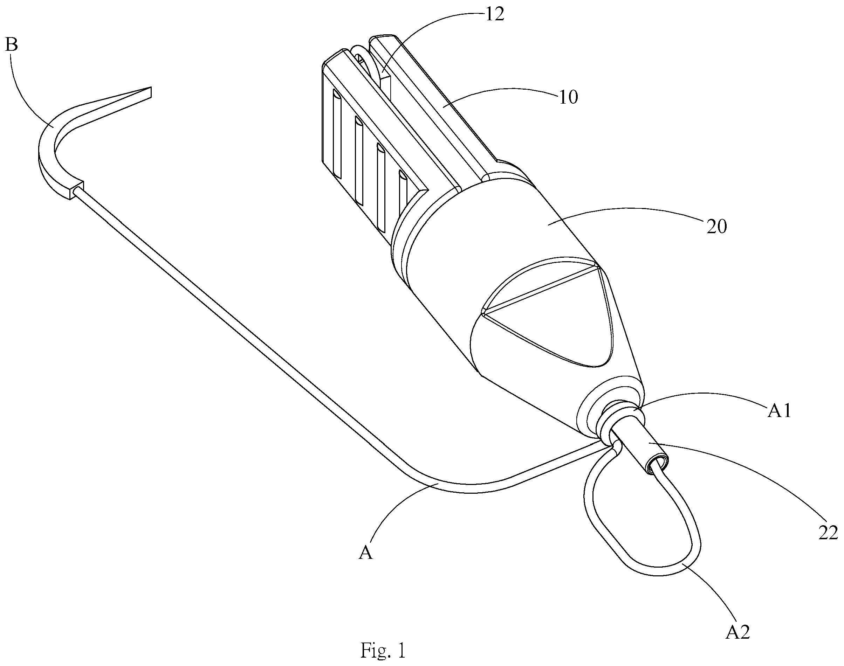

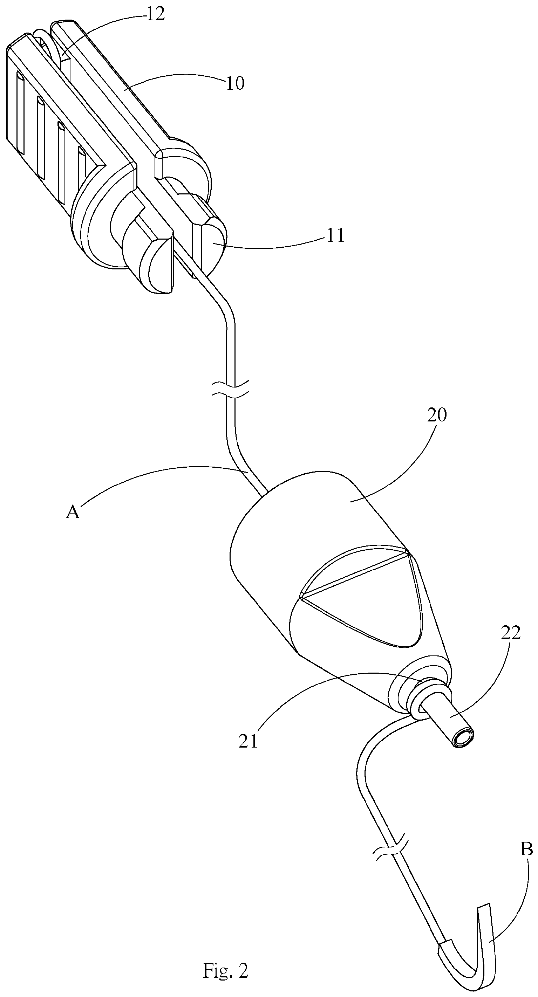

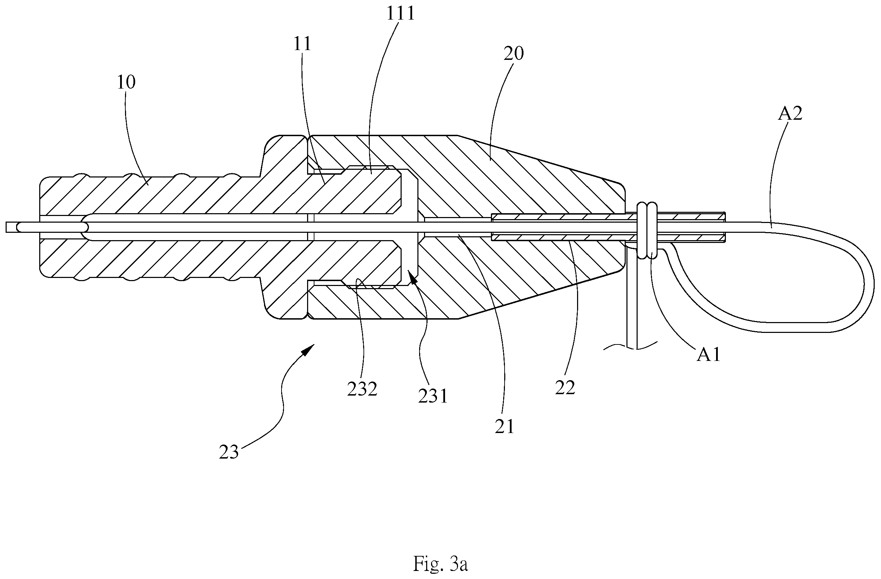

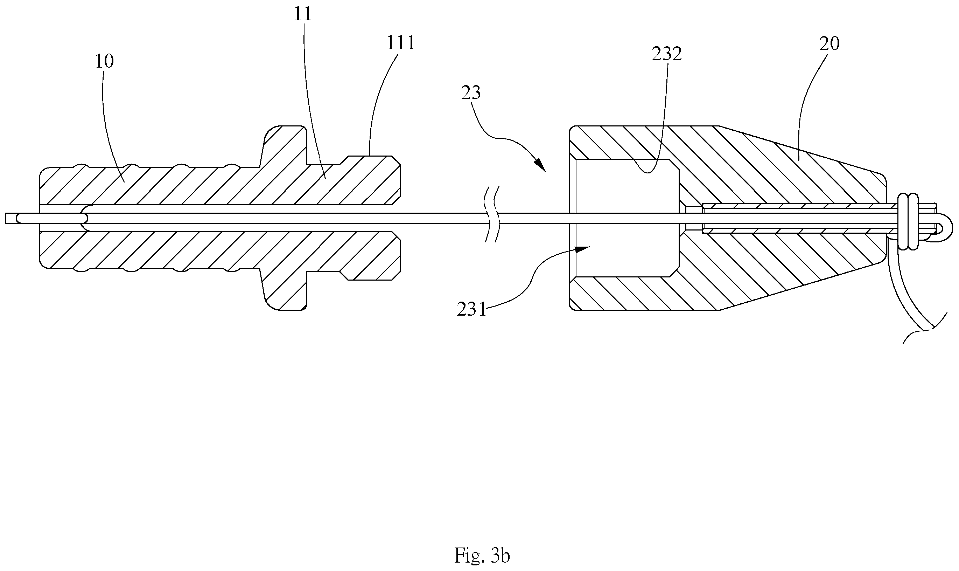

The present invention further described in detail below in conjunction with the drawings:
Refer to Figs. 1 to 3b, a first preferred embodiment of a capsule knotting module in accordance with the present invention is illustrated and comprises an operating member 10 and a knotting member 20. The operating member 10 is formed on a rear end of the capsule knotting module, and the knotting member 20 is formed on a front end of the capsule knotting module. The knotting member 20 comprises a through hole 21 defined through the knotting member 20, and a suture A extends through the through hole 21. An operating end of the suture A is connected to a part of the operating member 10. Means for connecting the operating end of the suture A and a part of the operating member 10 is not limited in the present invention. Methods, such as gluing or binding are all included in the scope of the present invention. In the embodiment, the operating member 10 comprises a pull rod 12 arranged diametrically, and the operating end of the suture A is bound on the pull rod 12.

A shaft 22 is a hollow tubular, is disposed in the through hole 21, and protrudes from the front end of the through hole 21 of the knotting member 20. An end opposite the operating end of the suture A is defined as a knotting end. After the knotting end of the suture A extends through the through hole 21 and the shaft 22, the knotting end extends toward and is exposed from the front end of the capsule knotting module.

The knotting end of the suture A exposed of the capsule knotting module is wrapped around an outer circumference of the shaft 22 to form a pre-knot A1 and a sliding loop A2 formed between the shaft 22 and the pre-knot A1. A surgical needle B is connected to an end of the knotting end of the suture A for suturing and fixing a tissue wound.

In the first embodiment, at least a part of the shaft 22 is securely disposed in the through hole 21. When the capsule knotting module is in use, a user aligns a front end of the knotting member 20 with an intended suture site and the surgical needle B is passed through the intended suture site, and the surgical needle B with the suture A/the knotting end extends through the sliding loop A2. Furthermore, during suturing process, a second suture different from the suture A can be applied to suture the intended suture site and extends through the sliding loop A2. After suturing process, the user moves the operating member 10 away from the knotting member 20, the operating end of the suture A is pulled, thereby the sliding loop A2 is shrunk until at least a part of the sliding loop A2 abutting against the outer circumference of the shaft 22 and tighten around the shaft 22. Then the operating member 10 is continuously pulled and/or the surgical needle B is simultaneously pulled, tensions generated on two ends of the suture A to make the pre-knot A1 move forward and escape from the shaft 22. Thus, the sliding loop A2 and the suture A extending through the sliding loop A2 are tied together to form a secure knot.

In another preferred embodiment, the shaft 22 can be slid along the through hole 21, and a length of the shaft 22 protruding from a front end of the through hole 21 can be adjusted according to a position of the shaft 22 relative to the through hole 21. The at least a part of the shaft 22 protruding from the through hole 21 is defined as a tying portion 221. When the operating member 10 is kept being pulled away from the knotting member 20, the sliding loop A2 is constricted and urge the shaft 22 to move inside the through hole 21. Thus, the length of the tying portion 221 is gradually shorten. When the tying portion 221 is completely moved into the through hole 21, the pre-knot A1 is slipped off the shaft 22 and the sliding loop A2 and the suture A extending through the sliding loop A2 are tie together to form the secure knot.

In another embodiment, the knotting member 20 comprises a connecting portion 23 for being detachably assembled with the operating member 10 to prevent the operating member 10 from being pulled unexpectedly. The way for assembling the operating member 10 with the connecting portion 23 is not limited in the present invention, methods such as concave-convex matching or providing a connector detachably arranged between the connecting portion 23 and the operating member 10 are all included in the scope of the present invention.

In this embodiment, a cavity 231 is defined in a rear end of the knotting member 20 and is served as the connecting portion 23, and a groove 232 is annually defined in at least a part of an inner wall of the cavity 231. The operating member 10 comprises two connecting ribs 11 protruding from the front end of the operating member 10 and spaced with each other. A farthest distance between two connecting ribs 11 is smaller than a diameter of the cavity 231. The two connecting ribs 11 on the operating member 10 can be inserted detachably into the cavity 231 in the knotting member 20. Preferably, the cavity 231 in the knotting member 20 communicates with the rear end of the through hole 21. The two connecting ribs 11 protrude from the front end of the operating member 10 from the two ends of the pull rod 12. The operating end of the suture A extends through the through hole 21, the cavity 231, and a gap between the two connecting ribs 11 in sequence and is tied with the pull rod 12.

A raised part 111 diametrically protrudes from a front end of each connecting rib 11 at a position corresponding to the groove 232. When the two connecting ribs 11 are inserted into the cavity 231, the raised parts 111 are held in the groove 232 to keep the operating member 10 from being separated from the knotting member 20 with an engagement between the raised parts 111 and the groove 232.

During the operation, to disengage the operating member 10 from the knotting member 20, a force is applied to the two connecting ribs 11 to move the connecting ribs 11 toward each other. Thus, the engagement between the raised parts 111 and the groove 232 can be released, such that the operating member 10 can be disengaged from the knotting member 20. The capsule knotting module of the present invention has a simple structure and can be designed in various suture size as needed. It can be miniaturized for fit a minimally invasive surgery, and assisting operators in completing suturing and knotting procedures seamlessly in narrow spaces, optimizing the surgical time and the quality of knotting.

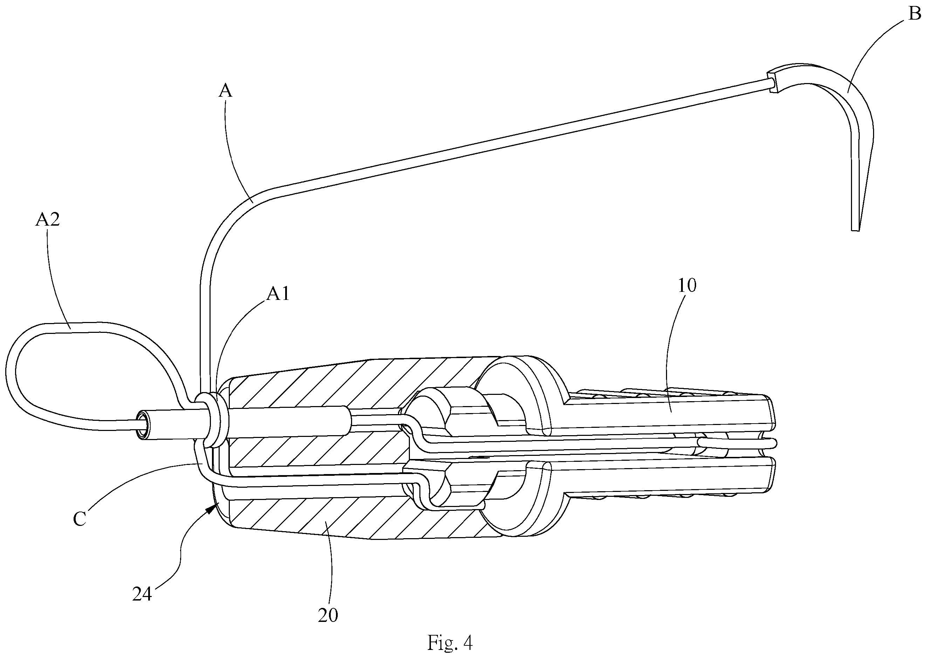

With reference to Fig. 4, a second preferred embodiment is illustrated. A difference between the second embodiment from the first embodiment is that the front end of the knotting member 20 further includes a limiting hole 24, a limiting suture C extends toward the front end of the knotting member 20 and out from the limiting hole 24. A limiting loop C1 is formed on at least a part of the limiting suture C protruding out of the limiting hole 24. At least a part of the limiting loop C1 is hooked with the pre-knot A1 and/or the sliding loop A2 to prevent the pre-knot A1 and the sliding loop A2 from being separated from the shaft 22 under unexpected conditions.

In this embodiment, the through hole 21 and the limiting hole 24 are parallel to each other and formed through the knotting member 20. The rear end of the through hole 21 and the limiting hole 24 communicate with the cavity 231. The limiting suture C is folded and held in the limiting hole 24, and two ends of the limiting suture C extend toward the rear end of the limiting hole 24 and into the cavity 231. The limiting loop C1 formed by being folded extends toward the front end of the limiting hole 24 and is hooked with the pre-knot A1. When the operating member 10 is assembled with the connecting portion 23 of the knotting member 20 in a concave-convex manner, at least a part of the operating member 10 compress the two ends of the limiting suture C between the connecting portions 23. Thus, the limiting suture C only can be released after the operating member 10 is separated from the knotting member 20, and the pre-knot A1 is released at the same time and the secure knot is formed subsequently.

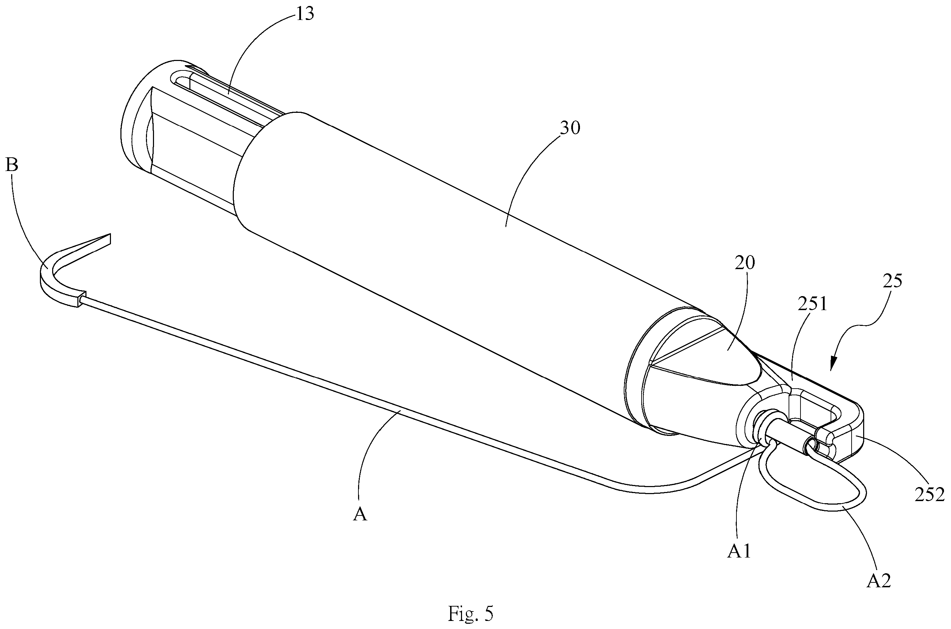

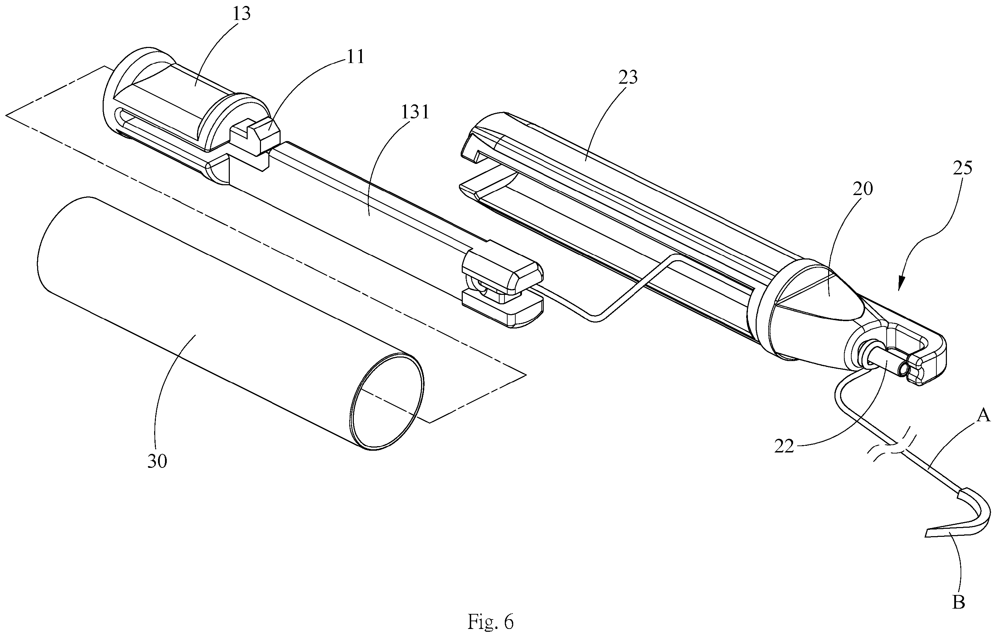

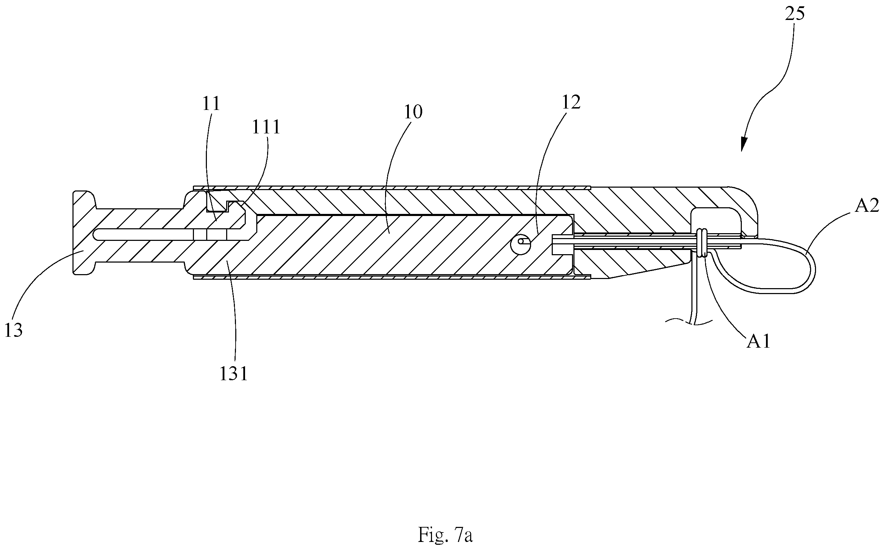

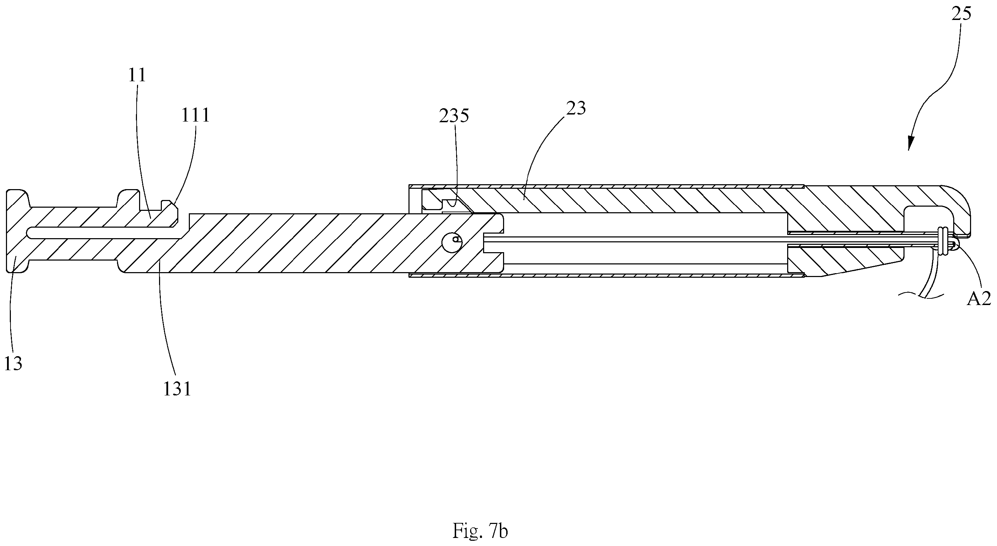

Further referring to a third preferred embodiment provided in Figs. 5 to 7b, the capsule knotting module comprises the operating member 10, the knotting member 20, and a housing 30. The housing 30 covers at least a part of the front end of the operating member 10 and at least a part of the rear end of the knotting member 20. The difference from the aforementioned embodiments is that the capsule knotting module is rod-shaped, this can minimize the use of suture A while increasing the length of the capsule knotting module. The capsule knotting module can be designed into different sizes according to requirements and optimize cost-effectiveness.

The operating member 10 is rod-shaped. The pull rod 12 is formed on the front end of the operating member 10 and is connected with the operating end of the suture A. The front end of the operating member 10 may slide relative to the knotting member 20 in the housing 30, and the rear end of the operating member 10 protrudes out of the housing 30 to form an operating portion 13 applied for enabling the user to hold the operating portion 13 to pull the operating member 10 to slide relative to the knotting member 20.

The capsule knotting module further comprises the connecting portion 23. The position of the connecting portion 23 is not limited, may be located between the operating member 10 and the knotting member 20, or between the operating member 10 and the housing 30. Structures or components for preventing the operating member 10 from being separated from the knotting member 20 unexpectedly are all included in the scope of the present invention.

Preferably, the connecting portion 23 is formed on the rear end of the knotting member 20 and extends into and is held in the housing 30. At least a part of the connecting portion 23 and at least a part of the front end of the operating member 10 are overlapped with each other in the housing 30. The connecting portion 23 may be the cavity 231 or a track, such that the front end of the operating member 10 is slidable in the housing 30 relative to the connecting portion 23.

In this embodiment, the connecting portion 23 is extends toward the rear end to a position adjacent to the operating portion 13. A notch 235 is defined in a surface of the connecting portion 23 corresponding to the operating member 10. An extending arm 131 and the connecting rib 11 protrude from the front end of the operating portion 13. The extending arm 131 and connecting rib 11 are spaced from each other. The connecting rib 11 is arranged at a position corresponding to the connecting portion 23.

Wherein, a front end of the extending arm 131 extends into the housing 30 and is connected with the pull rod 12. The front end of the connecting rib 11 extends into the housing 30 and the raised part 111 is raised toward to the notch 235. Accordingly, the operating member 10 is kept from be disengaged from the knotting member 20 with the engagement between the raised part 111 and the notch 235. To disengage the operating member 10 from the knotting member 20, a pressure is applied to the connecting rib 11 to deform the connecting rib 11 and to move the connecting rib 11 toward the extending arm 131, such that the raised part 111 can be escaped from the cavity 231 to release the concave-convex engagement relationship.

In another embodiment, an outer diameter of the knotting member 20 is tapered from the rear end to the front end so as to help the user to confirm a current position of the front end of the knotting member 20. Thus, the capsule knotting module can be prevented from being interfered with the user's field of vision when aligning the knotting member 20 with the intended suture site.

In another embodiment, a stop portion 25 is formed on the front end of the knotting member 20, and a holding space 26 is defined between the front end of the knotting member 20 and the stop portion 25. Preferably, the stop portion 25 comprises an extension section 251 extending from the front end of the knotting member 20 at a surface of the front end of the knotting member 20, and a stopping section 252 protrudes diametrically from the front end of the extension section 251 and corresponds to the through hole 21 in position. The holding space 26 is defined between the through hole 21, the extension section 251, and the stopping section 252, and a size of the holding space 26 can be defined by the length of the extension section 251.

When not in use, the length of the tying portion 221 of the shaft 22 is equal to the length of holding space 26. Preferably, at least a part of the outer circumference of the rear end of the tying portion 221 can be supported by the stopping section 252 to enhance the structural strength of the capsule knotting module. Furthermore, the outer circumference of the rear end of the tying portion 221 abuts with an end surface of the stopping section 252 to help the user to align the capsule knotting module with the intended suture site. Accordingly, the shaft 22 can be kept from being in directly contact with the suture A and being contaminated with blood or tissue fluid. At the same time, while the stop portion 25 is supported by the shaft 22, the pre-knot A1 can be effectively prevented from being slipped from the shaft 22 unexpectedly.

It should be noted. in the embodiment of that the shaft 22 is securely disposed in the through hole 21 and during the process of forming the secure knot, sufficient tension is required for pulling the operating member 10 and/or the surgical needle B. Thus, the pre-knot A1 can push the stop portion 25 to be deformed to allow the pre-knot A1 to slip away from the shaft 22. During this process, the stop portion 25 can prevent the pre-knot A1 from being slipped off from the shaft 22 in advance, and the secure knot is prevented from being in an undesirable position or being of poor quality and easy to be loosened.

Even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. A capsule knotting module, **characterized in that** the capsule knotting module comprises:
an operating member (10) arranged at a rear end of the capsule knotting module;
a knotting member (20) arranged at a front end of the capsule knotting module and having a through hole (21) formed through the knotting member (20), and a shaft (22) extending through the through hole (21) and protruding from the front end of the capsule knotting module; and
a suture (A) having two ends, one of the two ends of the suture (A) connected to a part of the operating member (10), and the other end of the suture (A) extending through the through hole (21) and the shaft (22) and extending toward and exposed from the front end of the capsule knotting module, wherein
at least a part of a knotting end of the suture (A) is wrapped around an outer circumference of the shaft (22) and forms a pre-knot (A1), and a sliding loop (A2) is formed between the shaft (22) and the pre-knot (A1); and
a size of the sliding loop (A2) is varied according to a distance between the operating member (10) and the knotting member (20).

2. The capsule knotting module as claimed in claim 1, wherein the knotting member (20) comprises a connecting portion (23) detachably connected with the operating member (10).

3. The capsule knotting module as claimed in claim 2, wherein the connecting portion (23) comprises a cavity (231) defined in a rear end of the knotting member (20), wherein a groove (232) is defined annually in an inner wall of the cavity (231), and the operating member (10) comprises two connecting ribs (11) extending toward the cavity (231) and spaced from each other, each connecting rib (11) having a raised part (111) diametrically protruding from a front end of the connecting rib (11) at a position corresponding to the groove (232) and engaged with the groove (232) in a concave-convex manner.

4. The capsule knotting module as claimed claim 2, wherein the knotting member (20) comprises a stop portion (25) formed on the knotting member (20), and the stop portion (25) comprises an extension section (251) formed on a surface of the knotting member (20) and extending toward the front end of the capsule knotting module and a stopping section (252) protruding in a diametrical direction of the through hole (21) from a front end of the extension section (251), wherein a rear end of the shaft (22) protruding out from the through hole (21) abuts with an end surface of the stopping section (252).

5. The capsule knotting module as claimed in any one of claims 1 to 4, wherein the operating member (10) comprises a pull rod (12) formed diametrically on the operating member (10), one of the ends of the suture (A) is bound on the pull rod (12).

6. The capsule knotting module as claimed in claim 5, wherein the two connecting ribs (11) protrude respectively from two ends of the pull rod (12) and extend toward the front end of the capsule knotting module, the suture (A) extends through the through hole (21), the cavity (231), and a space between the two connecting ribs (11) and is bound on the pull rod (12).

7. The capsule knotting module as claimed in claim 2 or 4 further comprising a housing (30) covering at least a part of a front end of the operating member (10) and at least a part of a rear end of the knotting member (20), wherein
a pull rod (12) is formed on the front end of the operating member (10), one of the ends of the suture (A) is bound on the pull rod (12), an operating portion (13) is formed on a rear end of the operating member (10) and protrudes out of the housing (30), and the operating portion (13) is connected with the pull rod (12) by an extending arm (131); and
the connecting portion (23) is formed on the rear end of the knotting member (20) at a position adjacent to the operating portion (13), at least a part of the connecting portion (23) is overlapped with at least a part of the front end of the operating member (10) in the housing (30), a front end of the operating portion (13) is slidable relative to the connecting portion (23) in the housing (30), and a notch (235) is defined in a surface of the connecting portion (23) corresponding to the operating member (10), wherein
the operating member (10) comprises a connecting rib (11) protruding from the operating portion (13), the extending arm (131) and the connecting rib (11) are spaced from each other, and a raised part (111) protrudes from the connecting rib (11) on a front end extending into the housing (30) and facing the groove (232).

8. The capsule knotting module as claimed in any one of claims 1 to 4, wherein a tying portion (221) is defined on at least a part of the shaft (22) protruding from the through hole (21), and a length of the tying portion (221) is varied according to a relative position of the shaft (22) and the through hole (21), wherein:
during an operation, at least a part of the suture (A) extends through the sliding loop (A2), and the sliding loop (A2) is pulled to be shrunk and abuts with the outer circumference of the shaft (22);
the sliding loop (A2) continues to be shrunk and pushes the shaft (22) to move into the through hole (21) to decrease the length of the tying portion (221); and
when the shaft (22) is completely moved inside the through hole (21), and the pre-knot (A1) is detached from the shaft (22) and is tied with the sliding loop (A2) and at least a part of the suture (A) to form a secure knot.

9. The capsule knotting module as claimed in any one of claims 1 to 4, wherein at least a part of the shaft (22) is securely held in the through hole (21), wherein
during an operation, at least a part of the suture (A) extends through the sliding loop (A2), and the sliding loop (A2) is pulled to be shrunk and abuts with the outer circumference of the shaft (22);
the operating member (10) is continuously pulled to make the pre-knot (A1) to move toward the front end of the capsule knotting module until the pre-knot (A1) is detached from the shaft (22); and
the pre-knot (A1) is detached from the shaft (22) and is tied with the sliding loop (A2) and at least a part of the suture (A), and a secure knot is formed.

10. The capsule knotting module as claimed in any one of claims 1 to 4, wherein the knotting end of the suture (A) is connected with a surgical needle (B).
